# EUROPEAN PATENT APPLICATION

(11) **EP 3 878 831 A1**
(43) Date of publication of application: **15.09.2021**
(21) Application number: 20162198.4
(22) Date of filing: 10.03.2020
(51) Int. Cl.: C07C 29/141, B01J 21/08, B01J 23/889, B01J 27/16, B01J 35/10, B01J 37/02, B01J 37/18

(54) **IMPROVED NICKEL-COPPER-MANGANESE-CATALYST FOR THE PREPARATION OF ALCOHOLS BY HYDROGENATION OF THE CORRESPONDING ALDEHYDES AND KETONES**

(71) Applicant: BASF SE, 67056 Ludwigshafen am Rhein (DE)
(72) Inventor: STOCK, Christoph, 67056 Ludwigshafen am Rhein (DE); PAPP, Rainer, 67056 Ludwigshafen am Rhein (DE); HEIDEMANN, Thomas, 67056 Ludwigshafen am Rhein (DE); ZUEND, Stephan, Hayward, CA 94541 (US)
(74) Representative: BASF IP Association

(57) **Abstract**

The present invention relates to an improved supported nickel-copper-manganese-catalyst which is characterized by the presence of a phosphor compound for the preparation of alcohols by hydrogenation of the corresponding aldehydes and ketones. Such supported catalysts exhibit a superior yield and selectivity.

## Description

The present invention relates to an improved supported nickel-copper-manganese-catalyst for the preparation of alcohols by hydrogenation of the corresponding aldehydes and ketones which is characterized by a phosphorous content of at least 0.2% b.w. referred to the total mass of the nickel, copper and manganese of the catalyst. The invention also relates to a method of preparing such supported catalysts and to the method of hydrogenation of aldehydes and ketones for the preparation of the corresponding alcohols by the means of such supported catalysts.

The catalytic hydrogenation of aldehydes or ketones to form saturated alcohols under various conditions of pressure and temperature has been known for a long time. The catalysts used are the metals and mixtures of the same conventionally used for hydrogenation, if desired with an addition of other catalysts, such as metal oxides.

Numerous variants of this process are generally known. Usually, the aldehyde is hydrogenated in the liquid phase over the hydrogenation catalyst at temperatures from 100 to 220°C and hydrogen pressure from 8 to 300 bar. To counteract the formation of ethers and condensation products, which is undesirable, the reaction is usually carried out in the presence of water. Nevertheless, the formation of other by-products usually requires the subsequent distillation of the crude alcohol, even if pure aldehydes are used as the starting materials.

DE2628987A presented a supported catalyst for the hydrogenation of alkanals, wherein the active mass of the catalyst used comprises from 40 to 80% by weight of nickel, from 10 to 50% by weight of copper and from 2 to 10% by weight of manganese. The hydrogenation process using this catalyst produced highly pure alkanols. Also, this catalyst turned out to exhibit a long-lasting life compared to conventional supported catalysts with nickel and molybdenum as active components.

Further improvement of the process regarding the purity of the resulting alkanols was achieved by adding a base of an alkali or alkali earth metal, such as NaOH or KOH to the hydrogenation feed (DE10024542A). DE10024542A also discloses a variation of the catalyst of DE2628987A which is characterized by active mass comprising sodium oxide in addition to the metals nickel, copper and manganese (example 7).

To increase the yield of alcohol and also further reduce the purification effort, catalysts for the hydrogenation of alkanals to the corresponding alkanols are desired which are characterized by a further improved selectivity.

Now, it has been found that the already highly selective nickel-copper-manganese-catalysts can be further improved by adding phosphorous to the active mass of the catalyst. This finding contrasts with the teaching of WO95/14648A, where for a conventional bimetallic nickel-molybdenum-catalyst it is described that the selectivity to the alcohol can be increased by lowering the phosphorous content of the catalyst.

Accordingly, this invention provides a new hydrogenation nickel-containing supported catalyst having an active mass comprising from 40 to 80% b.w. of nickel, from 10 to 50% b.w. of copper and from 2 to 10% b.w. of manganese referred to the total mass of nickel, copper and manganese, which is characterized by a phosphorous content, calculated as atomic phosphor, of at least 0.2% b.w. referred to the total mass of the nickel, copper and manganese of the catalyst. Usually, the phosphorous content, calculated as atomic phosphor, is in the range from 0.2 to 3.0% b.w., preferably in the range from 0.5 to 2.5% b.w., and more preferably in the range of 1.0 to 2.0% b.w. referred to the total mass of the nickel, copper and manganese of the catalyst.

The active mass components nickel, copper and manganese of the supported catalyst may be present in form of chemical compounds, preferably as oxides, or completely or partially in metallic form. The latter is the active form of the supported catalyst.

The phosphorous content of the supported catalyst may be present in form of any phosphorous compound or in any form of elementary phosphor. Preferably the phosphorous content is present completely or partially in form of phosphor(V) compounds, such as phosphoric acid, salts thereof (e.g. sodium or potassium phosphate) or phosphor(V) oxide, or in form of elementary phosphor, or mixtures thereof. More preferably the phosphorous content is present completely or partially in form of phosphoric acid or phosphor(V) oxide or elementary phosphor, or mixtures thereof.

In a preferred embodiment of the invention, the active mass of the catalyst comprises at least one alkali or alkali earth oxide, more preferably at least one alkali oxide, most preferably sodium oxide. The active mass of the catalyst can comprise up to 5% b.w., preferably up to 3% b.w. of such alkali or alkali earth oxide in total referred to the total mass of the nickel, copper and manganese. Preferably, the total content of such further alkali or alkali earth oxide is in the range from 0.1% to 3.0% b.w. , more preferably in the range from 0.5 to 2.5% b.w., and most preferably in the range of 1.0 to 2.0% b.w., referred to the total mass of the nickel, copper and manganese. In a particular embodiment, the active mass of the catalyst comprises sodium oxide in the range from 0.1% to 3.0% b.w., more preferably in the range from 0.5 to 2.5% b.w., and most preferably in the range of 1.0 to 2.0% b.w., referred to the total mass of the nickel, copper and manganese.

The solid catalyst support may be any chemically substantially inert material, such as silica, alumina, silica/alumina, titania, zirconia, zeolites, clay materials and combinations thereof. Usually the active mass of the catalyst is supported on inert oxides are used, preferably aluminum oxide, titanium dioxide, silicon dioxide, or zirconium dioxide. The support is provided in a suitable form, e.g. particles, powder, or shaped as monoliths, structured packings, tablets, extrudates, or spheres. Advantageously, the support is a porous material to allow for a large surface area. In general, the support material is treated with soluble compounds of the active mass components, or alternatively these are coprecipitated, kneaded together or otherwise combined with precursor of the support material. The preferred method is to absorb the active mass components on the solid support material, calcine and reduce the product. Typically, the supported catalyst contains, for example, from 3 to 50% b.w., preferably from 10 to 40% b.w., more preferably from 15 to 30% b.w. (expressed as metal) of the active mass components nickel, copper and manganese.

The BET surface area of the supported catalyst is typically between 50 to 300 m²/g, preferably between 100-200 m²/g. The BET surface area as used herein is the value that can be measured by determining the amount of nitrogen adsorbed at 77 K and P/Po of approximately 0.3 and assuming a nitrogen cross sectional area of 16.2 A², after degassing the catalyst sample at 180°C in accordance with the standard DIN ISO 9277-2014. The density of the supported catalyst is usually in the range from 500 to 1000 g/L and more preferably in the range from 600 to 800 g/L.

The pore volume of the supported catalyst is typically in the range from 0.3 to 0.8 mL/g and more preferably in the range from 0.4 to 0.7 mL/g, as determined via mercury porosimetry in accordance with the standard DIN ISO 15901-1:2016.

The supported catalysts of the invention may be manufactured, for example, by impregnating the support with an aqueous solution of soluble salts of nickel, copper and manganese (e.g. nickel nitrate, copper nitrate and manganese nitrate), an aqueous solution of a soluble phosphate salt or phosphoric acid and optionally an aqueous solution of one or more soluble alkaline or alkaline earth salts, preferably sodium salts (e.g. sodium nitrate) containing the phosphorous and the metals in proportion to achieve the above percentages in the active mass, then drying the impregnated material and repeating this process until the desired amount of active mass has been applied to the support. The impregnated material is then heated, e.g. in air at a temperature from about 500 to 600°C (calcination).

After such calcination and before being used for the hydrogenation reaction, the nickel, copper and manganese oxides of the supported catalyst are then reduced to the active metal form, e.g. in the presence of hydrogen at a temperature in the range from 280 to 300°C. The reduction can be carried out in the hydrogenation reactor but in general it is more economical to use a separate apparatus for the reduction. Usually a stream of nitrogen and hydrogen is applied to the supported catalyst at a pressure of 1 to 10 bara, and the ratio of hydrogen is continuously increased up to 100% during this hydrogenation step. After cooling down to ambient temperature, the surface of the catalyst material can be passivated to allow better handling of the catalyst in the presence of air, e.g. by applying a mixture of nitrogen and air, starting with a very low air content (e.g. 0.1 vol-%) and being increased slowly up 100 vol-%, while the temperature of the catalyst should not exceed a temperature of 50°C, preferably of 35°C.

Accordingly, the present invention also provides a method for preparing the supported catalyst of the invention, comprising the steps of
(i) impregnating the support with an aqueous solution of soluble salts (preferably nitrates) of nickel, copper and manganese, an aqueous solution of a soluble phosphate salt or phosphoric acid and optionally an aqueous solution of one or more soluble alkaline or alkaline earth salts (preferably nitrates), containing the phosphorous and the metals in proportion to achieve the above percentages in the active mass,
(ii) drying the impregnated material of step (i),
(iii) optionally repeating steps (i) and (ii) until the desired amount of active mass has been applied to the support, and
(iv) calcinating the impregnated material of step (ii) or (iii) at a temperature in the range from about 500 to 600°C, to convert the metal salts to the corresponding oxides.

In a particular embodiment, the method for preparing the supported catalyst of the invention also comprises the subsequent step of
(v) reducing the nickel, copper and manganese oxides of the calcinated material of step (iv) completely or partially to the active metal form, whereas such reduction is carried out preferably in the present of hydrogen at a temperature in the range from 280 to 300°C.

According to the present invention there is provided a method for the production of a saturated alcohol by contacting liquid hydrogenation feed, which comprises at least one aldehyde or ketone, with a hydrogen-containing gas in the presence of the supported catalyst of the invention. In general, elevated temperatures, for example in the range from 100 to 300°C, preferably in the range from 110 to 250°C, and in particular in the range from 120 to 200°C, and pressures in the range from 1 to 700 bara, preferably in the range from 5 to 300 bara, and particularly preferably in the range from 30 to 50 bara, are set. The hydrogenation process according to the invention can be carried out either batchwise or continuously, for example with the aid of tubular reactors or reactor cascades. The catalyst bed generally rests on a suitable retention element in the reactor. The hydrogenation reactor can be operated either by the pool or trickle method. The hydrogenation process according to the invention is preferably carried out in a reactor cascade, in particular a cascade comprising two to five reactors.

The supported catalyst is generally loaded with from 0.01 to 2, preferably, with from 0.1 to 1 and in particular with from 0.2 to 0.5 L of aldehyde/L of supported catalyst per hour.

The addition of water to the hydrogenation feed is possible in the process according to the invention but is not necessary. The hydrogenation feed generally contains traces of water, for example in the range from 1 ppm to 1% b.w., which have been introduced by the starting materials in the preceding synthesis steps or formed by condensation reactions. These traces of water are unimportant for the hydrogenation process according to the invention.

The liquid hydrogenation feed can consist of one or more undiluted aldehydes or ketones. However, in a particular embodiment the aldehydes or ketones are employed as a solution in an inert diluent. Examples of suitable inert diluents are hydrocarbons, ethers, such as diethyl ether, or alcohols. The diluents are particularly preferably alcohols, in particular the alcohol which is the hydrogenation product of the aldehyde to be hydrogenated. In a preferred embodiment, part of the hydrogenation product is recycled for this purpose and mixed with the aldehyde to be hydrogenated. If used, the inert diluent is preferably used in an amount of from 0.1 to 100 parts b.w., in particular from 1 to 50 parts b.w. and particularly preferably from 5 to 20 parts b.w., based on 1 part b.w. of aldehyde and ketone employed. If the hydrogenation is carried out adiabatically, i.e. with removal of the heat of reaction by the reaction product, the amount of inert diluent used is advantageously such that the temperature gradient over the bed of the granular catalyst does not exceed a temperature of 40°C. If, by contrast, the hydrogenation reactor is operated isothermally, the proportion of inert diluent in the hydrogenation feed can be selected virtually as desired.

In a preferred embodiment of the hydrogenation process, the hydrogenation feed comprises an amount of an alkali metal or alkali earth metal salt-like base with a basic anion (pKs value of the corresponding acid of the basic anion is >2, preferably >4, in particularly, >8). Suitable basic anions are hydroxide, carbonate, hydrogencarbonate, phosphate, amide, hydride; alkoxides, in particular C1-C4 -alkoxides, such as methoxide, ethoxide, n- and iso-propoxide and butoxide; phenoxide, carboxylates, such as acetate or benzoate; carbanions, such as butyl, cyclopentadienyl or phenyl. In general, hydroxide or carbonate is preferred. Advantageous salt-like bases are, in particular, alkali metal hydroxides and/or carbonates, such as lithium carbonate, potassium carbonate, sodium carbonate, lithium hydroxide, sodium hydroxide and potassium hydroxide. In general, sodium hydroxide and/or potassium hydroxide are preferred. However, sodium alkoxides and/or potassium alkoxides, such as the methoxide or ethoxide, or the alkoxide of the alcohol which is the hydrogenation product of the aldehyde present in the hydrogenation feed can also be employed with particular advantage. The salt-like bases are generally added to the hydrogenation feed in an amount which corresponds, on neutralization equivalent basis, to the range from 0.1 to 2000 ppm b.w., preferably from 0.1 to 1000 ppm b.w., in particular from 0.1 to 100 ppm b.w., particular preferably from 0.5 to 50 ppm b.w. and in particular from 1 to 20 ppm b.w., based on the aldehyde and ketone present in the hydrogenation feed, of potassium hydroxide. In the case of monovalent basic anions, a molar amount of salt-like base which corresponds to the stated amount of potassium hydroxide is used, and in the case of divalent basic anions, half the molar amount is used. It is also possible to add mixtures of different bases.

The resulting alcohols of the hydrogenation process can be further worked up by means of distillation.
The aldehydes and ketones to be hydrogenated according to the invention are not subject to any restrictions in principle. Preferably, aldehydes are employed for the hydrogenation according to the invention. The aldehydes which can be employed are preferably aliphatic C2- to C20-, in particular C3- to C15-aldehydes, which may be straight-chain or branched and may additionally contain C=C double bonds in the molecule. Suitable aldehydes which are of particular economic importance are, for example, acetaldehyde, propanal, n-butanal, isobutyraldehyde, crotonaldehyde, pentanal, 2-ethyl-4-methylpentenal, hexanal, 2-ethylhexanal, 2-ethylhexenal, 2-propylheptanal, 2-propylheptenal, octanal, nonanal, decanal, decenal, benzaldehyde, 2-phenylacetaldehyde, cinnamaldehyde, p-chlorobenzaldehyde, p-methoxybenzaldehyde and the hydroformylation products of trimeric and tetrameric propylene and dimeric and trimeric butene.

The hydrogen-containing gas preferably comprises more than 80 mol-% of hydrogen; in particular, it essentially consists of hydrogen. The hydrogen-containing gas can be passed over the fixed bed of the supported catalyst in co-current or counter-current to the hydrogenation feed. It is preferably passed in co-current. The amount of hydrogen-containing gas fed in is advantageously such that from 1.0 to 1.15 times the stoichiometrically necessary amount of hydrogen is available.

The amount of supported catalyst is preferably such that during the hydrogenation, active metal (sum of metallic nickel, copper and manganese) in the range from 40 to 100 g is available per mole of aldehyde, assuming a residence time of one hour.

### Examples

### Example 1

### Preparation of the catalyst

A catalyst according to the invention (catalyst A) was prepared by mixing 1.22 kg of nickel nitrate solution (13.5% b.w. of nickel), 0.35 kg of copper nitrate solution (15.5% b.w. of copper), 0.11 kg of manganese nitrate solution (12.5% b.w. of manganese), 59 g of sodium nitrate solution (4.1% b.w. of sodium) and 15 g of phosphoric acid having a concentration of 75% (23.7% b.w. of phosphor) and immersing 100 g of silica support material (SiO₂ strands having a diameter of 4 mm, a SiO₂ content of >99%, a BET surface area of 150 m²/g and a pore volume of 0.97 ml/g; support material type D11-10 by BASF SE) in this aqueous mixture for 30 min. After dripping off excessive solution, the impregnated material was dried over night at a temperature of 120°C and subsequently calcinated at a temperature of 600°C for a period of 2 h. This process of immersing, drying and calcination was repeated with the catalyst material a second time. The resulting catalyst material contained 21.3% b.w. of nickel, calculated as NiO, 7.3% b.w. of copper, calculated as CuO, 2.0% b.w. of manganese, calculated as Mn₃O₄, 0.35% b.w. of sodium, calculated as Na₂O, and 1.2% b.w. of phosphor, calculated as H₃PO₄, remainder SiO₂. This corresponds to an active mass with a weight ratio of 70 : 24 : 6 for the active mass components nickel: copper: manganese, and with 1.5% b.w. of sodium oxide and 1.6% b.w. of atomic phosphor, both referred to the total of weight of nickel, copper and manganese.

For comparison, a second catalyst (catalyst B) was prepared by the same procedure but without addition of phosphoric acid.
A further catalyst according to the invention (catalyst C) was prepared accordingly but applying Siliperl® AF125 (SiO₂ beads having a diameter of 3 to 6 mm, a SiO₂ content of >99%, a BET surface area of 270 m²/g and a pore volume of 0.82 ml/g; support material type Siliperl® AF125 by BASF SE) as silica support material. The resulting catalyst material contained 17.6% b.w. of nickel, calculated as NiO, 6.0% b.w. of copper, calculated as CuO, 1.7% b.w. of manganese, calculated as Mn₃O₄, 0.29% b.w. of sodium, calculated as Na₂O, and 1.0% b.w. of phosphor, calculated as H₃PO₄, remainder SiO₂. This corresponds to an active mass with a weight ratio of 70 : 24 : 6 for the active mass components nickel: copper: manganese, and with 1.5% b.w. of sodium oxide and 1.6% b.w. of atomic phosphor, both referred to the total of weight of nickel, copper and manganese.

After cooling down to ambient temperature, the catalysts (catalyst A, B and C) were installed in reduction devices where the active mass was reduced by a mixture of nitrogen and hydrogen at a temperature of 250°C and at a pressure of 1 to 10 bara for 24 h. During this reduction step, the ratio of hydrogen within the stream of nitrogen and hydrogen was increased continuously from 1 to 100 vol-%. After cooling down to ambient temperature again, the surface of the catalyst material was passivated to allow better handling of the catalyst in the presence of air, by applying a mixture of nitrogen and air, starting with an air content of 0.1 vol-% and being increased slowly up 100 vol-%, in a way that the temperature of the catalyst does not exceed 35°C.

### Example 2

### Catalytic hydrogenation of n-butanal to n-butanol

A continuous flow hydrogenation apparatus was charged with 190 ml of activated catalyst A or B, respectively. The reaction conditions were 35 bar, 150°C and the feed to recycle ratio was 1:12. n-butyric aldehyde from a hydroformaylation was the hydrogenation feed. To the feed 15 ppm KOH, dissolved in butanol were added. At a load of 0.42 kg/l*h and 150°C, 35 bar, n-butyric aldehyde was hydrogenated to n-butanol with a selectivity of 99.3% (by-products: 0.07% dibutyl ether, 0.04 % b.w. butylbutyrat, 0.15% b.w. ethylhexanediol and 0.13% b.w. dibutylbutylacetal) for catalyst A, and with a selectivity of 97.8% (by-products: 0.65% b.w. dibutyl ether, 0.04 % b.w. butylbutyrat, 0.36% b.w. ethylhexanediol and 0.78% b.w. dibutylbutylacetal) for catalyst B.

By adding phosphorous to the active mass of the catalyst, the selectivity of the catalytic hydrogenation is significantly increased.

### Example 3

### Catalytic hydrogenation of iso-butanal to iso-butanol

The hydrogenation of Example 2 was carried out in the same way but with iso-butyric aldehyde from the hydroformaylation in the reaction feed instead of n-butyric aldehyde. Using catalyst A, iso-butanol was prepared with a selectivity of 99.32% (by-products: 0.23% b.w. diisobutyl ether, 0.02% b.w. isobutylisobutyrat, 0.28% b.w. diisobutylisobutylisobutylacetal).

### Example 4

### Catalytic hydrogenation of n-pentanal to n-pentanol

The hydrogenation of Example 2 was carried out in the same way but with valeric aldehyde from the hydroformaylation in the reaction feed instead of n-butyric aldehyde. Using catalyst A, n-pentanal was prepared with a selectivity of 99.32% (by-products: 0.23% b.w. dipentanol ether, 0.02% b.w. pentylpentanoat, 0.28% b.w. acetal).

## Claims

1. A supported catalyst having an active mass comprising from 40 to 80% b.w. of nickel, f
rom 10 to 50% b.w. of copper and from 2 to 10% b.w. of manganese referred to the total mass of nickel, copper and manganese,
which is **characterized by** a phosphorous content of at least 0.2% b.w. referred to the total mass of the nickel, copper and manganese of the catalyst.

2. The supported catalyst of claim 1, whereas the active mass further comprises up to 5% b.w. of alkali or alkali earth oxide in total referred to the total mass of the nickel, copper and manganese.

3. The supported catalyst of claim 2, whereas alkali or alkali earth oxide is sodium oxide.

4. A method for preparing the supported catalyst of any of claims 1 to 3, comprising the steps of
(i) impregnating the support with an aqueous solution of soluble salts of nickel, copper and manganese, an aqueous solution of a soluble phosphate salt or phosphoric acid and optionally an aqueous solution of one or more soluble alkaline or alkaline earth salts, containing the phosphorous and the metals in proportion to achieve the above percentages in the active mass,
(ii) drying the impregnated material of step (i),
(iii) optionally repeating steps (i) and (ii) until the desired amount of active mass has been applied to the support, and
(iv) calcinating the impregnated material of step (ii) or (iii) at a temperature in the range from about 500 to 600°C, to convert the metal salts to the corresponding oxides.

5. The method of claim 4, which comprises the subsequent step of
(v) reducing the nickel, copper and manganese oxides of the calcinated material of step (iv) completely or partially to the active metal form.

6. A method for the production of a saturated alcohol by contacting a liquid hydrogenation feed, which comprises at least one aldehyde or ketone, with a hydrogen-containing gas in the presence of the supported catalyst of any of claims 1 to 3.

7. The method of claim 6, whereas the reaction temperature is set in the range from 100 to 300°C.

8. The method of claim 6 or 7, whereas the pressure is set in the range from 1 to 700 bar.

9. The method of any of claims 6 to 8, whereas hydrogenation feed comprises an aldehydes selected from the group consisting of acetaldehyde, propanal, n-butanal, isobutyraldehyde, crotonaldehyde, pentanal, 2-ethyl-4-methylpentenal, hexanal, 2-ethylhexanal, 2-ethylhexenal, 2-propylheptanal, 2-propylheptenal, octanal, nonanal, decanal, decenal, benzaldehyde, 2-phenylacetaldehyde, cinnamaldehyde, p-chlorobenzaldehyde, p-methoxybenzaldehyde and the hydroformylation products of trimeric and tetrameric propylene and dimeric and trimeric butene.

10. The method of any of claims 6 to 9, whereas the hydrogenation feed comprises an amount of an alkali metal or alkali earth metal salt-like base with a basic anion.
